# EUROPEAN PATENT SPECIFICATION

(11) **EP 3 303 554 B1**
(45) Date of publication and mention of the grant of the patent: **24.06.2020**
(21) Application number: 16723246.1
(22) Date of filing: 02.05.2016
(51) Int. Cl.: C12N 5/00, C07D 311/62, C07H 17/07, C07C 13/62

(54) **DEEP EUTECTIC SOLVENTS AND/OR IONIC LIQUIDS AS FEED MEDIA**
TIEFE EUTEKTISCHE LÖSUNGSMITTEL UND/ODER IONISCHE FLÜSSIGKEITEN ALS ZUFUHRMEDIEN
SOLVANTS EUTECTIQUES PROFONDS ET/OU LIQUIDES IONIQUES UTILISÉS COMME MILIEUX D'ALIMENTATION

(30) Priority: 29.05.2015 EP 15001612
(43) Date of publication of application: 11.04.2018
(73) Proprietor: Merck Patent GmbH, 64293 Darmstadt (DE)
(72) Inventor: RAYNER, Michael Howard, 64342 Seeheim-Jugenhei (DE); VON HAGEN, Joerg, 64319 Pfungstadt (DE); SIECK, Jochen, Bastian, 64291 Darmstadt (DE); KNACK, Claudia, 64287 Darmstadt (DE)
(86) International application number: PCT/EP2016/000716
(87) International publication number: WO 2016/192829

(56) References cited:
- WO-A1-2011/134921
- MAAN HAYYAN ET AL: "In Vitro and In Vivo Toxicity Profiling of Ammonium-Based Deep Eutectic Solvents", PLOS ONE, vol. 10, no. 2, 13 February 2015 (2015-02-13), page e0117934, XP055286119, DOI: 10.1371/journal.pone.0117934
- VENKATA NANCHARAIAH Y ET AL: "Alkyl-methylimidazolium ionic liquids affect the growth and fermentative metabolism ofsp", BIORESOURCE TECHNOLOGY, ELSEVIER BV, GB, vol. 102, no. 11, 16 March 2011 (2011-03-16), pages 6573-6578, XP028480911, ISSN: 0960-8524, DOI: 10.1016/J.BIORTECH.2011.03.042 [retrieved on 2011-03-21]
- NANCHARAIAH Y V ET AL: "Hormetic effect of ionic liquid 1-ethyl-3-methylimidazolium acetate on bacteria", CHEMOSPHERE, vol. 128, 19 February 2015 (2015-02-19), pages 178-183, XP029148033, ISSN: 0045-6535, DOI: 10.1016/J.CHEMOSPHERE.2015.01.032
- YUNTAO DAI ET AL: "Natural deep eutectic solvents as new potential media for green technology", ANALYTICA CHIMICA ACTA, vol. 766, 1 March 2013 (2013-03-01), pages 61-68, XP055286799, NL ISSN: 0003-2670, DOI: 10.1016/j.aca.2012.12.019
- V. Fischer: "Properties and Applications of Deep Eutectic Solvents and Low-Melting Mixtures", , 1 January 2015 (2015-01-01), pages 1-146, XP055287000, Retrieved from the Internet: URL:http://epub.uni-regensburg.de/31832/1/ Doktorarbeit_Veronika_Fischer_04_05_15.pdf [retrieved on 2015-07-08]
- RONJA MUELLER ET AL: "Improved fed-batch bioprocesses using chemically modified amino acids in concentrated feeds", BMC PROCEEDINGS, BIOMED CENTRAL LTD, LONDON UK, vol. 7, no. Suppl 6, 4 December 2013 (2013-12-04), page P46, XP021170344, ISSN: 1753-6561, DOI: 10.1186/1753-6561-7-S6-P46
- CLAUDIA KNACK ET AL: "Manufacturing ultra-concentrated liquid feeds: Transitioning the aqueous solubility barrier of the feed amino acids cysteine and tyrosine", BMC PROCEEDINGS, vol. 9, no. Suppl 9, 14 December 2015 (2015-12-14), page P54, XP055286191, London UK ISSN: 1753-6561, DOI: 10.1186/1753-6561-9-S9-P54

## Description

The present invention relates to feed media comprising deep eutectic solvents and/or ionic liquids.

Cell culture media in aqueous solution can provide an environment which supports and maintains the growth of cells and/or maintains a desired physiological cellular condition adventitious to the targeted production of certain products, so called target molecules.

Cell culture media comprise of a complex mixture of components, sometimes more than one hundred different components, depending on the type of organism whose growth and/or targeted physiological status shall be supported.

The first cell culture media that were developed were complex media consisting of diverse mixtures of components which were very poorly chemically defined, poorly characterized and difficult to manufacture with a consistent quality, such as plasma, serum, embryo extracts, and/or other biological extracts or peptones. A major advance was thus made with the development of chemically defined media. Chemically defined media often comprise of, but are not exclusively limited to, amino acids, vitamins, saccharides, metal salts, antioxidants, chelators, growth factors, buffers, hormones, and many more substances known to those expert in the art.

Some cell culture media are offered as sterile aqueous liquids. The disadvantage of liquid cell culture media is their reduced shelf life and difficulties for shipping and storage. As a consequence, many cell culture media are presently offered as finely milled dry powder mixtures. These are designed, often with other supplements, for supplying cells with a substantial nutrient base for growth and/or production of biopharmaceuticals from said cells and/or used as a feed to supply cells when specific nutrients are used up.

For the final use in cell culture, the dry powder mixtures are dissolved in water and/or aqueous solutions and are added to the cell culture in the dissolved state because it is typically desirable to have components for use in cell culture in a liquid form due to the inherent disadvantages of solids, for example difficulty of sterile addition and/or turbidity of solids added due to slow dissolution. In addition, solids are more difficult to dose to systems, for example to bioreactor systems containing biological entities.

Often the pure nutrient components are solids in and around room temperature. Consequently, such components need to be dissolved in solvents in order to provide a practicable liquid form. This has the disadvantage, for many poorly soluble components, that large volumes are required to add the desired component.

As a consequence large amounts of solvents, such as water, have to be added to the system with its inherently limited volume. The solvent quickly fills the bioreactor and dilutes the product, and hence reduces, amongst other aspects, the overall economic efficiency of the process. This means that the efficacy with respect to the space and time consumption is higher if the bioreactor volume can be kept as low as possible, i.e. the contents remain as concentrated as possible and do not get diluted.

Therefore, it would be favourable to find a way to provide feed media in a liquid but highly concentrated form in order to be able to add the necessary feed components without adding too much volume to the bioreactor and thereby minimizing the dilution effect on its contents.

It has been found that deep eutectic solvents and/or ionic liquids can be used as highly concentrated liquid feeds for cell culture in bioreactors. Deep eutectic solvents and/or ionic liquids are per se liquid and do not need to be dissolved by the addition of a solvent which has no other effect on the cell culture than diluting it. At least one component of the deep eutectic solvent and/or ionic liquid is, favourably, a nutrient component which is needed to supply cells when specific nutrients become limiting or less than optimal to support a targeted optimal physiological condition.

The present invention is thus directed to a liquid feed medium comprising a deep eutectic according to claim 1.

In a preferred embodiment, the liquid feed medium is liquid at or below 100 °C, preferably at or below 50 °C, most preferred it is liquid at or below 35 °C, especially between 20 and 35 °C. In any case it is added to the bioreactor in which the cell culture is performed in the liquid state, that means at a temperature at which it is liquid.

In another embodiment, the liquid feed medium comprises cysteine and/or tyrosine. The amino acids are typically a part of the deep eutectic solvent.

In another embodiment, the liquid feed medium comprises other components which are not part of the deep eutectic solvent and which are dissolved in the deep eutectic solvent.

The present invention is further directed to a process for cell culture comprising the following steps:
a) Providing a bioreactor with cells in a liquid cell culture medium
b) Adding to said liquid cell culture medium a liquid feed medium comprising a deep eutectic solvent according to the present invention

In a preferred embodiment, the liquid feed medium added in step b) has a temperature below 100 °C, preferably below 50 °C, most preferred it has a temperature between 20 and 35 °C.

In another embodiment, the liquid feed medium comprises cysteine and/or tyrosine.

In another embodiment, the liquid feed medium comprises other components which are not part of the ionic liquid or deep eutectic solvent which are dissolved in the ionic liquid and/or deep eutectic solvent.

In one embodiment, the cells in the bioreactor are stem cells, eukaryotic cells, prokaryotic cells, bacteria, archaea, yeasts, fungi, insect cells or algae.

The liquid feed medium comprises less than 50% (w/w), preferably less than 20% of water, most preferred less than 10% of water.

The present invention is further directed to the use of a liquid feed medium according to the present invention as a nutrient feed.
Figures 1 to 4 show data of cell culture experiments performed with the media and the process of the present invention. Figure 1 shows the viability and osmolality of a parental CHO-S fed batch process. Figure 2 shows the lactate and glutamate content and the pH of the same process. Further details can be found in Example 2a).
Figure 3 shows the qMAB and the viability of a transfected CHO-S cell culture producing a monoclonal antibody. Figure 4 shows the osmolality and the pH of the same cell culture. Further details can be found in Example 2b).

A cell culture is any setup in which cells are cultured. A cell culture is for example used to e.g. produce cells (such as stem cells or cellular compartments), or to produce target molecules like pharmaceuticals, recombinant proteins, viruses, vaccines, enzymes, metabolites, hormones, lipids, colour agents, nucleic acids, etc.

A cell culture is performed in a bioreactor. A bioreactor is any unit suitable for the culture of cells, such as a container, vessel, bag, flask or tank in which cells can be cultured. A bioreactor is typically sterilized prior to use. Incubation is typically performed under suitable conditions such as suitable temperature, osmolality, aeration, agitation, etc. A person skilled in the art is aware of suitable incubation conditions for supporting or maintaining the growth/culturing of cells.

A cell culture medium according to the present invention is any mixture of components which maintains and/or supports the *in vitro* growth of cells and/ or supports a particular physiological state. It might be a complex medium or a chemically defined medium. The cell culture medium can comprise all components necessary to maintain and/or support the *in vitro* growth of cells or be used for the addition of selected components in combination with further components that are added separately. Examples of cell culture media according to the present invention are full media, also called base media, which comprise all components necessary to maintain and/or support the *in vitro* growth of cells as well as media supplements or feed media.

Typically, the cell culture media according to the invention are used to maintain and/or support the growth of cells and/ or support a particular physiological state in a bioreactor.

During the growth of cells in a bioreactor or fermenter it is often economically important to maintain the growth phase and/or the production phase on-going for a long period of time (days, weeks, months). Thus, often, some initial components supplied to the cells as base medium when the cell culture is started become exhausted. Such components cannot always be supplied initially at higher concentration due to solubility problems and/or because they have a toxic and/or otherwise negative effect on the manufacturing process. Thus, such components need to be added later either continuously or discontinuously during the running axenic culturing process into the bioreactor. Such components are, for example cysteine, or it's biologically relevant and/or active derivatives as well as tyrosine since they are essential to many cells and/or manufacturing processes.

A feed medium is thus a cell culture medium that is added to a cell culture continuously or discontinuously at a later stage of the cell culture process. That means it is not the cell culture medium with which the cells are cultivated to start the cell culture process in the bioreactor. A feed medium is typically added to a bioreactor one or several times in the course of the process. A feed medium is typically added as a nutrient feed and/or as an osmolarity modulator and/or as a pH regulator and/or to support growth of cells and/or to support the production of target molecules.

A feed medium typically comprises less components than a full medium. It may only comprise one or two components. Typically it comprises 2 to 10 components.

The cell culture media, especially the feed media, according to the present invention can be designed to be suitable to grow or maintain/support the growth many different kinds of organism, e.g. prokaryotic cells like bacterial cells or eukaryotic cells like yeast, fungi, algae, plant, insect or mammalian cells or archaea. The cells can be normal cells, immortalized cells, diseased cells, transformed cells, mutant cells, somatic cells, germ cells, stem cells, precursor cells or embryonic cells, any of which may be established or transformed cell lines or obtained from natural sources.

A mammalian cell culture medium is a mixture of components which maintain and/or support the *in vitro* growth of mammalian cells. Examples of mammalian cells are human or animal cells, preferably CHO cells, COS cells, I VERO cells, BHK cells, AK-1 cells, SP2/0 cells, L5.1 cells, hybridoma cells, insect cells or human cells.

Preferably the cell culture media, especially the feed media, according to the present invention, are chemically defined cell culture media.

Chemically defined cell culture media are cell culture media comprising of chemically well characterized 'defined' raw materials. This means that the chemical composition of all the chemicals used in the media is known. The chemically defined media do not comprise of chemically ill-defined yeast, animal or plant tissues; they do not comprise feeder cells, serum, extracts or digests or other components which may contribute chemically poorly defined proteins and/or peptides and/or hydrolysates to the media. Chemically undefined or poorly defined chemical components are those whose chemical composition and structure is not well known, are present in poorly defined and varying composition or could only be defined with enormous experimental effort - comparable to the evaluation of the chemical composition and structure of a protein-digest from albumin or casein.

A powdered cell culture medium or a dry powder medium is a cell culture medium typically resulting from a milling process or a lyophilisation process. That means the powdered cell culture medium is typically a finely granular, particulate medium - not a liquid medium. The term "dry powder" may be used interchangeably with the term "powder;" however, "dry powder" as used herein simply refers to the gross appearance of the granulated material and is not intended to mean that the material is completely free of complexed or agglomerated solvent unless otherwise indicated. A powdered cell culture medium can also be a granulated cell culture medium, e.g. dry granulated by roller compaction.

A full cell culture medium to be used in the process of the present invention typically comprises at least one or more saccharide components, one or more amino acids, one or more vitamins or vitamin precursors, one or more salts, one or more buffer components, one or more co-factors and one or more nucleic acid components. It may also comprise recombinant proteins, e.g. rInsulin, rBSA, rTransferrin, rCytokines etc.

The media may also comprise sodium pyruvate, vegetable proteins, digests or extracts, fatty acids and/or fatty acid derivatives and/or pluronic product components (block copolymers based on ethylene oxide and propylene oxide) in particular Poloxamer 188 sometimes called Pluronic F 68 or Kolliphor P 188 or Lutrol F 68 and/or surface active components such as chemically prepared non-ionic surfactants. One example of a suitable non-ionic surfactant are difunctional block copolymer surfactants terminating in primary hydroxyl groups also called poloxamers, e.g. available under the trade name pluronic® from BASF, Germany. Such pluronic product components are in the following just called pluronic.

Saccharide components are all mono- or di-saccharides, like glucose, galactose, ribose or fructose (examples of monosaccharides) or sucrose, lactose or maltose (examples of disaccharides). Saccharide components may also be oligo- or polysaccharides.

Examples of amino acids according to the invention are the proteinogenic amino acids, especially the essential amino acids, leucine, isoleucine, lysine, methionine, phenylalanine, threonine, tryptophan and valine, as well as the non-proteinogenic amino acids such as D-amino acids.

Tyrosine means L- or D- tyrosine, preferably L-tyrosine.
Cysteine means L- or D-cysteine, preferably L-cysteine.

Amino acid precursors and analogues are also included.
Examples of vitamins are Vitamin A (Retinol, retinal, various retinoids, and four carotenoids), Vitamin B₁ (Thiamine), Vitamin B₂ (Riboflavin), Vitamin B₃ (Niacin, niacinamide), Vitamin B₅ (Pantothenic acid), Vitamin B₆ (Pyridoxine, pyridoxamine, pyridoxal), Vitamin B₇ (Biotin), Vitamin B₉ (Folic acid, folinic acid), Vitamin B₁₂ (Cyanocobalamin, hydroxycobalamin, methylcobalamin), Vitamin C (Ascorbic acid), Vitamin D (Ergocalciferol, cholecalciferol), Vitamin E (Tocopherols, tocotrienols) and Vitamin K (phylloquinone, menaquinones). Vitamin precursors and analogues are also included.

Examples of salts are components comprising inorganic ions such as bicarbonate, calcium, chloride, magnesium, phosphate, potassium and sodium or trace elements such as Co, Cu, F, Fe, Mn, Mo, Ni, Se, Si, Ni, Bi, V and Zn. Examples are copper(II) sulphate pentahydrate (CuSO₄·5 H₂O), sodium chloride (NaCl), calcium chloride (CaCl₂·2 H₂O), potassium chloride (KCl), iron(II)sulphate, sodium phosphate monobasic anhydrous (NaH₂PO₄), magnesium sulphate anhydrous (MgSO₄), sodium phosphate dibasic anhydrous (Na₂HPO₄), magnesium chloride hexahydrate (MgCl₂·6 H₂O), zinc sulphate heptahydrate (ZnSO₄·7 H₂O).

Examples of buffers are carbonate, phosphate, HEPES, PIPES, ACES, BES, TES, MOPS and TRIS.

Examples of cofactors are thiamine derivatives, biotin, vitamin C, NAD/NADP, cobalamin, vitamin B12, flavin mononucleotide and derivatives, glutathione, heme, nucleotide phophates and derivatives.

Nucleic acid components, according to the present invention, are the nucleobases, like cytosine, guanine, adenine, thymine or uracil, the nucleosides like cytidine, uridine, adenosine, guanosine and thymidine, and the nucleotides such as adenosine monophosphate or adenosine diphosphate or adenosine triphosphate.

Ionic liquids or liquid salts are ionic species which typically consist of an organic cation and an inorganic or organic anion. They do not contain any neutral molecules and are liquid below 100 °C, preferably below 50 °C, most preferred below 35 °C.

The area of ionic liquids is currently being researched intensively since the potential applications are multifarious. Review articles on ionic liquids are, for example, R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flussigkeiten - neue Losungen für die Übergangsmetallkatalyse" [Ionic Liquids - Novel Solutions for Transition-Metal Catalysis], Angew. Chem., 112 (2000), 3926-3945; T. Welton "Room temperature ionic liquids. Solvents for synthesis and catalysis", Chem. Rev., 92 (1999), 2071-2083 or R. Hagiwara, Ya. Ito "Room temperature ionic liquids of alkylimidazolium cations and fluoroanions", J. Fluorine Chem., 105 (2000), 221-227).

In general, all ionic liquids of the general formula K⁺A⁻ known to the person skilled in the art, in particular those which are miscible with water and non-toxic to the cells to be cultured, that means which are biologically compatible, are suitable in the disclosed method.

The anion A⁻ of the ionic liquid is preferably biologically compatible and e.g. selected from the group comprising OH⁻, halides, borates, phosphates, phosphites, phosphonates, phosphinates, silicates, cyanamide, thiocyanate, anions of carboxylic acids, carbonates, sulfates, sulphites, sulfonates, nitrate ([NO₃]⁻), anions of organic acids, or imides of the general formula [N(R_{f})₂]⁻ or of the general formula [N(XR_{f})₂]⁻, where Rf denotes partially or fully fluorine-substituted alkyl having 1 to 8 C atoms and X denotes SO₂ or CO.

Halides are for example Cl⁻, Br⁻, I⁻, preferably, Cl⁻, Br⁻.

Borates are for example BO₃³⁻, HBO₃²⁻, H₂BO₃⁻, R₂BO₃⁻, RHBO₃²⁻, B(OR)(OR)(OR)(OR)⁻, B(HSO₄)⁻, B(RSO₄)⁻, BF_{z},R^{F}_{4-z}⁻, with z = 0, 1, 2 or 3.

Phosphates are for example PO₄³⁻, HPO₄²⁻, H₂PO₄⁻ , R₂PO₄⁻, RPO4²⁻, HRPO₄⁻, PR^{F}_{y}F_{6-y}⁻, with y = 1, 2, 3, 4, 5 or 6.

Phosphites are for example PO₃³⁻, HPO₃²⁻ , H₂PO₃⁻, R₂PO₃⁻, RPO₃²⁻, HRPO₃⁻.

Carboxylic acids and carbonates are for example CH₃COO⁻, RCOO⁻, HCO₃⁻, CO₃²⁻, RCO₃⁻

Sulfates, sulphites, sulfonates are for example SO₄²⁻, HSO₄⁻, SO₃²⁻, HSO₃⁻, ROSO₃⁻, RSO₃⁻.

Phosphonates and phosphinates are for example RHPO₃⁻, R₂PO₂⁻, R₂PO₃⁻.

Silicates are for example SiO₄⁴⁻, HSiO₄³⁻, H₂SiO₄²⁻, R₂SiO₄²⁻, RSiO₄³⁻, R₃SiO₄⁻, H₂RSiO₄⁻.

In which R is each independently of another a non-fluorinated, partially fluorinated or perfluorinated straight-chain or branched alkyl group having 1 to 6 C atoms.

If R is perfluorinated it is preferably trifluoromethyl, pentafluoroethyl or nonafluorobutyl, very particularly preferably trifluoromethyl or pentafluoroethyl.

If R is non-fluorinated it is preferably methyl, ethyl, n-butyl, n-hexyl, very particularly preferably methyl or ethyl.

R^{F} is each independently of another a perfluorinated straight-chain or branched alkyl group having 1 to 6 C atoms. It is preferably trifluoromethyl, pentafluoroethyl or nonafluorobutyl, very particularly preferably trifluoromethyl or pentafluoroethyl.

Examples of anions of organic acids are pyruvate, lactate, acetate, citrate, butyrate, malate, oxalate and/or tartrate.

Preferably the anion is selected from anions of organic acids, halides, nitrates, sulfates, thiosulphates, phosphates, carbonates, sulfonates, hydroxides and carboxylates as described above. For example, the anion may be selected from chloride, acetate, trifluoroacetate, methanesulfonate, glycolate, benzoate, salicylate, (±)-lactate, (+) -lactate, (-) lactate, (+)-pantothenate, (±)-tartrate,. (+) -tartrate, (-) -tartrate, (±)-hydrogen tartrate, (+)-hydrogen tartrate, (-)-hydrogen tartrate, (±)-potassium tartrate, (+)-potassium tartrate, (-)-potassium tartrate, meso-tartrate, meso-1-hydrogen tartrate, meso-2-hydrogen tartrate, meso-1- potassium tartrate, meso-2-potassium tartrate. Another preferred anion is an organic carboxylate.

There are no restrictions per se with respect to the choice of the cation K⁺ of the ionic liquid. However, preference is given to biocompatible, organic cations such as: ammonium, phosphonium, uronium, thiouronium, guanidinium cations or heterocyclic cations.

Ammonium cations can be described, for example, by the formula (1)

[NRₐ₄]⁺ (1),

where
Rₐ in each case, independently of one another, denotes
H, where all substituents Rₐ cannot simultaneously be H,
OR', NR'₂, with the proviso that a maximum of one substituent Rₐ in formula (1) is OR', NR'₂,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more R may be partially or fully substituted by halogens, in particular -F and/or -CI, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' may be = H, non-, partially or perfluorinated C₁- to C₆-alkyl, C₃- to C₇-cycloalkyl, unsubstituted or substituted phenyl and X may be = halogen.

Phosphonium cations can be described, for example, by the formula (2)

[PR²₄]⁺ (2),

where
R² in each case, independently of one another, denotes H, OR' or NR'₂
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more R² may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R² which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = H, non-, partially or perfluorinated C₁- to C₆-alkyl, C₃- to C₇-cycloalkyl, unsubstituted or substituted phenyl and X = halogen.

Uronium cations can be described, for example, by the formula (3)

[(R³R⁴N)-C(=OR⁵)(NR⁶R⁷)]⁺ (3),

and thiouronium cations by the formula (4),

[(R³R⁴N)-C(=SR⁵)(NR⁶R⁷)]⁺ (4),

where
R³ to R⁷ each, independently of one another, denotes
hydrogen, where hydrogen is excluded for R⁵,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more of the substituents R³ to R⁷ may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R³ to R⁷ which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = H, non-, partially or perfluorinated C₁- to C₆-alkyl, C₃- to C₇-cycloalkyl, unsubstituted or substituted phenyl and X = halogen.

Guanidinium cations can be described by the formula (5)

[C(NR⁸R⁹)(NR¹⁰R¹¹)(NR¹²R¹³)]⁺ (5),

where
R⁸ to R¹³ each, independently of one another, denotes
hydrogen, -CN, NR'₂, -OR'
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more of the substituents R⁸ to R¹³ may be partially or fully substituted by halogens, in particular -F and/or -CI, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R⁸ to R¹³ which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = H, non-, partially or perfluorinated C₁- to C₆-alkyl, C₃- to C₇-cycloalkyl, unsubstituted or substituted phenyl and X = halogen.

Most preferred are cations composed of a quaternary nitrogen-based ion, preferably based on a nucleus selected from quaternary ammonium cations, hydroxylammonium cations, pyrazolium cations, imidazolium cations, triazolium cations, pyridinium cations, pyridazinium cations, pyrimidinium cations, pyrazinium cations and triazinium cations. The heterocyclic nucleus may be substituted at any carbon or nitrogen atom by any C1-C12 alkyl, alkenyl, alkoxy, alkenedioxy, allyl, aryl, arylalkyl, aryloxy, amino, aminoalkyl, thio, thioalkyl, hydroxyl, hydroxyalkyl, oxoalkyl, carboxyl, carboxyalkyl, haloalkyl or halide function including all salts, ethers, esters, pentavalent nitrogen or phosphorus derivatives or stereoisomers thereof. When required and where possible, any of these functions may include a functional group selected from the group consisting of alkenyl, hydroxyl, amino, thio, carbonyl and carboxyl groups.

Examples of hydroxylammonium cations are N-alkyl hydroxylammonium ions; N,N- dialkyl hydroxylammonium ions (for instance N,N-dimethyl, N-methyl-N-ethyl, N- methyl-N-propyl, N,N-diethyl, N-ethyl-N-propyl, N,N-dipropyl or N,N-dibutyl hydroxylammonium ions); N,N,N-trialkyl hydroxylammonium ions (for instance N,N,N-trimethyl, N-ethyl-N-methyl-N-propyl, N,N,N-triethyl or N,N,N-tripropyl hydroxylammonium ions); N-alkyl-N-hydroxyalkyl hydroxylammonium ions; N,N-dialkyl-N-hydroxyalkyl hydroxylammonium ions (for instance N,N-dimethyl-N-(2-hydroxyethyl) or N,N-dipropyl-N-(2-hydroxyethyl) hydroxylammonium ions); N-alkyl-O-alkyl hydroxylammonium ions (for instance N- ethyl-O-alkyl or N-alkyl-O-methyl or N-ethyl-O-methyl hydroxylammonium ions); O- alkyl-N,N-dialkyl hydroxylammonium ions (for instance O-methyl-N,N-dialkyl, O-.rho.ro.rho.yl-N,N-dialkyl, O-octyl-N,N-dialkyl, O-alkyl-N,N-diethyl or O-alkyl-N,N- dipropyl hydroxylammomum ions); O- alkyl-N,N,N-trialkyl hydroxylammonium ions, in particular O-methyl-N,N,N-trialkyl hydroxylammonium ions (for instance N,N,N,O-tetramethyl or N,N,N-triethyl-O- methyl hydroxylammomum ions); and 0-alkyl-N,N-dialkyl-N-hydroxyalkyl hydroxylammonium ions (for instance N,N,O-trimethyl-N-(2-hydroxyethyl), N5N- diethyl-N-(2-hydroxyethyl)-O-methyl orN,N-dipropyl-N-(2-hydroxyethyl)-O-methyl hydroxylammonium ions).

Particularly preferred cations are choline (the *N,N,N-*trimethylethanolammonium cation) and derivatives and/or trimethylglycine and/or other betaines. Suitable derivatives are for example 2-methyl-choline, ethers and esters of choline such as acetylcholine, lactylcholine, propinoylcholine, buturylcholine, or the methyl-, ethyl-, vinyl- or butyl-ether of choline and esters of betaine, Most preferred is the *N,N,N-*trimethylethanolammonium cation.

In one embodiment, the ionic liquid may also comprise ectoin or derivatives of ectoin ((S)-1,4,5,6-tetrahydro-2-methyl-4-pyrimidinecarboxylic acid).

Non-toxic, water soluble ionic liquids are for example disclosed in EP 1594974, EP 1805131, WO 2006/038013, WO 2007/036712 and WO 2007/063327.

Deep eutectic solvents are liquids having a melting point that is lower than the melting point of the two or more components that form the eutectic mixture. The components of the deep eutectic solvent (DES) typically interact with each other through hydrogen bond interactions. Examples of deep eutectic solvents are disclosed in WO 2011/15589 and Chem. Soc. Rev., 2012, 41, 7108-7146. Compared to ordinary solvents, deep eutectic solvents have a very low volatility and are typically non-flammable. They share a lot of characteristics with ionic liquids.

Typically deep eutectic solvents can be assigned to 4 groups: type 1 to type 4 DES:

| | |
|---|---|
| Type I | Quaternary ammonium salt + metal chloride |
| Type II | Quaternary ammonium salt + metal chloride hydrate |
| Type III | Quaternary ammonium salt + hydrogen bond donor |
| Type IV | Metal chloride hydrate + hydrogen bond donor |

For use in the present description type III DES comprising a quarternary ammonium salt and a hydrogen bond donor are especially preferred. Examples of hydrogen bond donors are alcohols, carboxylic acids, amines, amides like urea, acetamide or thiourea. Examples of alcohols are m-cresol, fructose, glycerol and ethylene glycole.

Examples of quarternary ammonium salts are choline salts, betaine, N-ethyl-2-hydroxy-N, N-dimethylethanaminiumchlorid, ethylammonium chloride, tetrabutylammonium chloride, triethylbenzylammonium chloride and acetylcholine chloride and derivatives thereof,
The hydrogen bond donor of the solvents is preferably selected from at least one naturally occurring organic acid, at least one naturally occurring mono- or dimeric sugar, sugar alcohol, amino acid, di or tri alkanol or betaine derivatives.

Said sugar or sugar alcohol may be selected from the group of sucrose, glucose, fructose, lactose, maltose, cellobiose, arabinose, ribose, ribulose, galactose, rhamnose, raffinose, xylose, sucrose, mannose, trehalose, mannitol, sorbitol, inositol, ribitol, galactitol, erythritol, xyletol and adonitol, and, as well as their phosphates.

The said organic acid may be selected from amino acids, malic acid, maleic acid, citric acid, lactic acid, pyruvic acid, fumaric acid, succinic acid, lactic acid, acetic acid, aconitic acid, tartaric acid, malonic acid, ascorbic acid, glucuronic acid, oxalic acid, neuraminic acid and sialic acids.

The DES preferably comprise quaternary ammonium salts like choline or betaine. An example of a DES is a mixture of choline chloride and urea in a 1:2 molar ratio. Other deep eutectic solvents of choline chloride are formed with malonic acid, citric acid, succinic acid, phenol and glycerol. Examples of DES formed with betaine are mixtures of betaine with urea, malonic acid or citric acid.

Preferably, the DES to be used in the present description comprise a quaternary ammonium salt and an amino acid.

Preferably, the DES to be used in the present description comprise choline or betaine or derivatives or salts thereof. Suitable derivatives are for example 2-methyl-choline, ethers and esters of choline such as acetylcholine, lactylcholine, propinoylcholine, buturylcholine, or the methyl-, ethyl-, vinyl- or butyl-ether of choline and esters of betaine, Most preferred is choline. Suitable examples are e.g. disclosed in J. Am. Chem. Soc. 2004, 126, 9142-9147. Typically the cholines are present in the form of the chloride or hydroxide.

The DES comprise an amino acid and/or derivatives thereof. Examples of suitable derivatives are inorganic ester derivatives such as in case of tyrosine and cysteine (S)-2-Amino-3-(4-phosphonooxy-phenyl)-propionic acid or salts thereof and (S)-2-amino-3-sulfosulfanylpropanoic acid or salts thereof.

Most preferred are the amino acids cysteine and/or tyrosine and their derivatives.

The DES to be used according to the present invention is comprised of choline and/or betaine and/or derivatives or salts thereof and one or more amino acids.

The gist of the present invention is to provide liquid feed media that are highly concentrated and comprise as little solvent like water as possible. It has been found that deep eutectic solvents and/or ionic liquids are perfectly suitable as feed media as they are liquids. Furthermore, one can choose the composition of the deep eutectic solvents or ionic liquids such that they are formed by at least one component which is required in the feed medium. That means, the solvent of the feed medium is not only used as a solvent but is part of the feed itself.

A liquid is an almost incompressible fluid that conforms to the shape of its container but retains an (almost) constant volume independent of pressure. As such, it is one of the four fundamental states of matter (the others being solid, gas, and plasma), and is the only state with a definite volume but no fixed shape. A liquid is a fluid.

In one embodiment the liquid feed media of the present invention are only formed by a deep eutectic solvent. A person skilled in the art knows how to make deep eutectic solvents or ionic liquids.

There are various methods to synthesize ionic liquids known to a person skilled in the art. One way of synthesizing ionic liquids is to use a one-way reaction, in which the desired ionic liquids are produced directly from their starting materials. Hereby, the cation and anion are formed together in the same working step.

As an alternative, an ionic liquid can be synthesized via two or more reaction steps. For this, typically, the cation is prepared as a salt with an easily changeable anion such as a halide anion. Afterwards an anion metathesis is performed. Anion metathesis can be realized in various ways in accordance with the available anion source and preference of the method leading to minimal degree of impurities as possible. Processes for the preparation of ionic liquids are described, for example, in P. Wasserscheid, T. Welton (Eds.), Ionic Liquids in Synthesis, Second Edition, WILEY-VCH, Weinheim, 2008.

Deep eutectic solvents can for example be prepared by mixing all components and treat them under elevated temperature. The temperature is dependent on the components. For DES comprising choline, a temperature between 100 and 150 °C is typically suitable. Preferably, the mixture is agitated during heating, e.g. by stirring, shaking etc.
The deep eutectic solvent is formed when the mixture becomes a clear liquid which is free of crystals. Typically, a mixing time between 30 and 60 minutes is suitable.
In case of DES comprising three or more components, it is also possible to form two or more binary mixtures each comprising two components of the envisaged DES and heat them to generate binary DES. The two or more binary DES are then mixed in a second step to form the DES comprising three or more components.

The deep eutectic solvent and/or the ionic liquid can be used directly as liquid feed medium. If the manufacturing process of the DES or ionic liquid does not provide a product with acceptably low bioburden level then the bioburden may be reduced to suitable levels by the application by, for example, of filtering, autoclaving, gamma sterilizing or using other techniques known by those skilled in the art. Autoclaving is preferred.

The DES and/or ionic liquid can also be used to solubilize further components such as saccharides like glucose, trace elements, vitamins, amino acids, pluronic, signal molecules like IPTG or insulin or others which are typically added in feed media.

To modify the viscosity of the liquid feed medium, a solvent, such as water or glycerol, preferably water, can be added. Typically the liquid feed medium does not comprise more than 50% of a solvent such as water (w/w). Preferably, it does not comprise more than 10% (w/w).

Preferably, the viscosity of the liquid feed medium should be such that it can be subjected to sterile filtration.

In a preferred embodiment, the liquid feed medium of the invention comprises choline or betaine. Those components can e.g. be present as a salt. Choline can for example be present as choline hydroxide, choline chloride, choline bicarbonate.

The liquid feed medium of the present invention comprises a deep eutectic solvent comprising choline. In a preferred embodiment, the liquid feed medium comprises a DES formed at least by choline or a choline derivative and an amino acid such as tyrosine or cysteine or a derivative.

Preferably, to form a DES comprising tyrosine, this component is added in the form of tyrosine HCI. Preferably, to form a DES comprising cysteine, this component is added in the form of cysteine HCI H₂O.

Especially preferred DES are made by or comprise the following mixtures:
- Cys HCl H₂O: ChCl, preferably between 0.75:1 and 1:2.5 (w/w)
- Tyr HCl: ChCl: H₂O, preferably Tyr HCl and ChCl have a ratio between 0.75:3 and 1.5:3 (w/w), most preferred 1:3 and the ratio between water and the mixture of Tyr HCl and ChCl is between 0.75:1 and 3:1, preferably between 3:3 and 3:5 (w/w)
- ChCl: Cys HCl H₂O: Tyr HCl H₂O (e.g. 6:1.5:1:10 (w/w))

The pH of the DES and/or the ionic liquid can be amended by adding suitable buffer components and/or by selection of the components and their specific salt forms, e.g. choline chloride versus choline hydroxide.

For use in cell culture, the liquid feed medium of the present invention is typically added to the cell culture at a later stage and in addition to the full medium used to start the cell culture.

The present invention is thus further directed to a process for cell culture comprising the following steps:
a) Providing a bioreactor with cells in a liquid cell culture medium according to claim 1
b) Adding to said bioreactor a liquid feed medium comprising a deep eutectic solvent according to the present invention

Then cells are further cultured in the bioreactor. Performing a cell culture is known to a person skilled in the art. This is typically done by incubating the cells under suitable conditions like pH, osmolality, temperature, agitation, aeration (oxygen/CO₂) etc. and the optional addition of feed media one or several times during the cell culture. Preferably, the cell culture is performed as fed-batch cell culture.

Fed-batch culture is a cell culture process where one or more nutrients (substrates) are fed (supplied) to the bioreactor during cultivation of the cells and in which the product(s) remain in the bioreactor until the end of the run. An alternative description of the method is that of a culture in which a base medium supports the initial cell culture and a feed medium is added to prevent nutrient depletion. The advantage of the fed-batch culture is that one can control concentration of fed-substrate in the culture liquid at arbitrarily desired levels.

Generally speaking, fed-batch culture is superior to conventional batch culture when controlling concentrations of a nutrient (or nutrients) affect the yield or productivity of the desired metabolite.

In a preferred embodiment, the liquid feed medium added in step b) has a temperature below 100 °C, preferably below 50 °C, most preferred it has a temperature between 20 and 35 °C.

The liquid feed medium of the present invention comprises amino acids, preferably cysteine and/or tyrosine.

In another embodiment, the liquid feed medium comprises other components which are not part of the deep eutectic solvent which are dissolved in the deep eutectic solvent.

In one embodiment, the cells in the bioreactor are stem cells, eukaryotic cells, prokaryotic cells, yeasts, fungi, insect cells or algae.

The liquid feed medium of the present invention comprises less than 50% (w/w), preferably less than 10% of water.

Preferably, the pH of the liquid feed medium is between pH 5 and 9, most preferred between pH 6 and 8.

The DES feed additions are scheduled based on the cell culture demand of the specific feed component. A typical addition scheme for a feed containing tyrosine and/or cysteine would start at culture day 3 and would be added every second day. The added amounts should be as low as possible, taking into account the high concentration of DES feeds, compared to typical aqueous feeds. Amounts may range from 0,01g to 0,2g per addition per feed, added to 30 mL cell culture (equals 1:3000 to 1:150 per feed per addition).

In a preferred embodiment, all liquid feed media that are added to the cell culture comprise a DES. Depending on the protocol of the cell culture, additional liquid feed is added one or several times in the course of the culture process. The composition of the liquid feed medium that is added can be identical each time it is added or different.

The present invention is further directed to the use of a liquid feed medium according to the present invention as a nutrient feed Also disclosed is the use of this liquid feed medium as an osmolarity modulator and/or as a pH regulator and/or to support growth of cells and/or to support the production of target molecules. Depending on the composition of the liquid feed medium one or several of the above mentioned effects can be reached by adding the liquid feed medium according to the present invention to a cell culture.

The liquid feed medium to be used in the process of the present invention offers an alternative to the addition of the feed components in their solid form. The liquid feed media of the present invention combine a high concentration with excellent dissolution properties. For the first time, highly concentrated feed media can be added to a cell culture. The volume of the cell culture is kept as low as possible without causing dilution by the addition of large volumes of a solvent. This leads to a cell culture with efficient cell growth, the cells can be kept at the desired optimal physiological state. Process parameters such as product yield, process speed and space/time consumption can be optimized. The cell culture process can be kept very stable and defined, e.g. as it is not necessary to add tyrosine and/or cysteine in a large basic feed volume.

It is possible to take one or several desired components, such as amino acids, with high melting points, for example cysteine or tyrosine, and which are not very soluble under standard conditions in biologically compatible solvents, for example water, and nevertheless to make a liquid containing nearly 50% amino acid by contacting them with at least a second solid, for example choline chloride.

Such mixtures can be further contacted with other components (solids, liquids or gases) in order to tune the properties of the mixture and to make it more desirable for its intended use. For example the physicochemical properties such as the viscosity of the deep eutectic solvent and/or an ionic liquid can be optimized by the addition of small amounts of water.
Thus, such mixtures may comprise between 10% and 100% DES.

The components of the deep eutectic solvent and/or an ionic liquid are selected from compounds that do not cause negative effects on the cellular growth. The non-toxic compounds choline and betaine are ideal in this respect.

The present invention is further illustrated by the following figures and examples, however, without being restricted thereto.

### Examples

The following examples represent practical applications of the invention.

### 1. DES preparation & characterisation

All components are weighed and mixed into beakers and treated under elevated temperature. Components are stirred with a suitable device, for example a magnetic stirrer or spatula until a clear, crystal free liquid is formed. Autoclavating improves the liquid formation and dissolution of remaining crystals of DES with Tyr HCl: Choline chloride. Furthermore, water supports DES formation and reduces viscosity. For pH measurement, water needs to be added.

Table 1 gives an overview about suitable combinations to form a DES to be used as liquid feed medium.
(n) = molar ratio (mol)
(m) = mass ratio (g)

**Table 1:**

| | **Composition** | **ratio** | **conditions** | **pH value** |
|---|---|---|---|---|
| **1** | Choline hydroxide : Choline chloride | 1:1 (n) | 30 min, 120 °C | 11.9 |
| **2** | Choline chloride : Choline bicarbonate | 1:1 (n) | 30 min, 120 °C | 15.1 |
| **3** | Choline bicarbonate: Choline hydroxide | 1:1 (n) | 30 min , 120 °C | 12.5 |
| **4** | Variant **1** : Tyr HCl | 10:1(m) | 5 min, 120 °C | - |
| **5** | Variant **1** : Tyr Disodiumsalt 2 H₂O | 10:1(m) | 5 min, 120 °C | - |
| **6** | Variant **1** : Tyr Disodiumsalt 2 H₂O: Cys HCl H₂O | 10:1:1 (m) | 5 min, 120 °C | 8.7 |
| **7** | Choline chloride: Glucose | 1:1 (m) | 30 min, 120 °C | |
| **8** | Variant **3** : Tyr Disodiumsalt 2 H₂O: Cys HCl H₂O | 10:1:1 (m) | 1 min. 120°C | 9.9 |
| **9** | Variant **3** : Tyr HCl: Cys HCl H₂O | 10:1:1 (m) | 1 min. 120 °C | 9.3 |
| **10** | Choline chloride : Tyr HCl : H₂O | 3:1:3 (n) | 30 min, 120°C | - |
| **11** | Choline hydroxide: Tyrosine | 3:1 (m) | 5 min, 120°C | - |
| **12** | Choline hydroxide : Tyr Disodiumsalt 2 H₂O | 3:1 (m) | 5 min, 120°C | - |
| **13** | Choline hydroxide : Tyr Disodiumsalt 2 H₂O: Cys HCl H2O | 3:1:1 (m) | 5min, 120°C | 12.4 |
| **14** | Choline chloride: Cys HCl H₂O | 1:1 (n) | 45min, 120°C | 1.2 |
| **15** | Choline chloride : Cys HCl H₂O | 2:1 (n) | 45min, 120°C | - |
| **16** | Choline Hydroxide: pTyr | 5:1 (n) | 5min, 120°C | 10.1 |

### 2. Application of DES in cell culture processes

Fed Batch Process Strategy using a standard feed (include 300 mM Cys HCI H₂O and 573 mM Tyr 2Na with pH 11. The stock solution yields concentrations of cysteine and tyrosine of 300 and 573 mM, respectively, which are subsequently diluted during feeding (Tab.2)) or DES feeds (10 and 14) according to the present invention is shown in Table 2:

**Table 2:**

| **Cultivation [d]** | **0** | **3** | **4** | **5** | **6** | **7** | **10** | **11** | **12** | **13** | **14** |
|---|---|---|---|---|---|---|---|---|---|---|---|
| **CHO 220 Feed (%v/v)** | | 3 | | 6 | | 6 | 6 | | | | 6 |

| **Glucose** | Monitor daily and maintain at 4-6 g/l | | | | | | | | | | |
|---|---|---|---|---|---|---|---|---|---|---|---|
| **Cys/Tyr (%v/v)** | | 0.3 | | 0.6 | | 0.6 | 0.6 | | | | 0.6 |
| **Cys DES [g]** | | 0.01 | | 0.017 | | 0.017 | 0.017 | | | | 0.017 |
| **Tyr DES [g]** | | 0.037 | | 0.07 | | 0.07 | 0.07 | | | | 0.07 |

The same molar amount of Cys and Tyr in DES and standard aqueous feed is added (theoretical calculation).

### Preparation:

Fed-Batch experiments are conducted in 35 mL medium with CHO cells (0.3 x 106 cells/mL) in 50 ml spin tubes, with feeding every second day, starting day 3. The same molar amount of Cys and Tyr in DES and standard aqueous feed is added. Glucose is fed as carbon source and maintained at 4-6 g/L. Every day, 1.5 mL cell suspension is taken for pH and osmolality measurements and for metabolite and antibody concentration determination. The experiments are typically stopped when viability drops below 80%.

### Results:

### a) Parental CHO-S (without production of antibody)

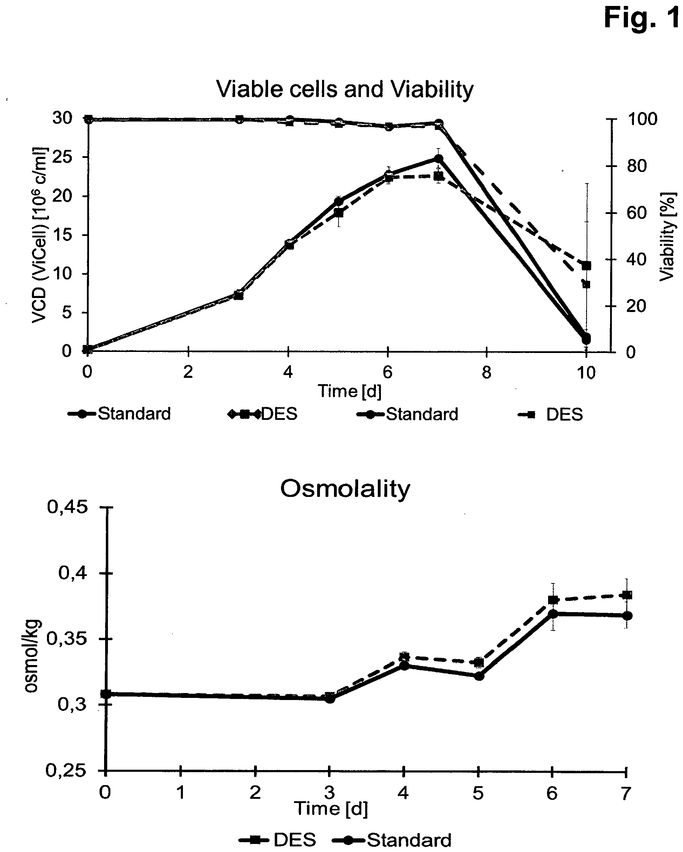

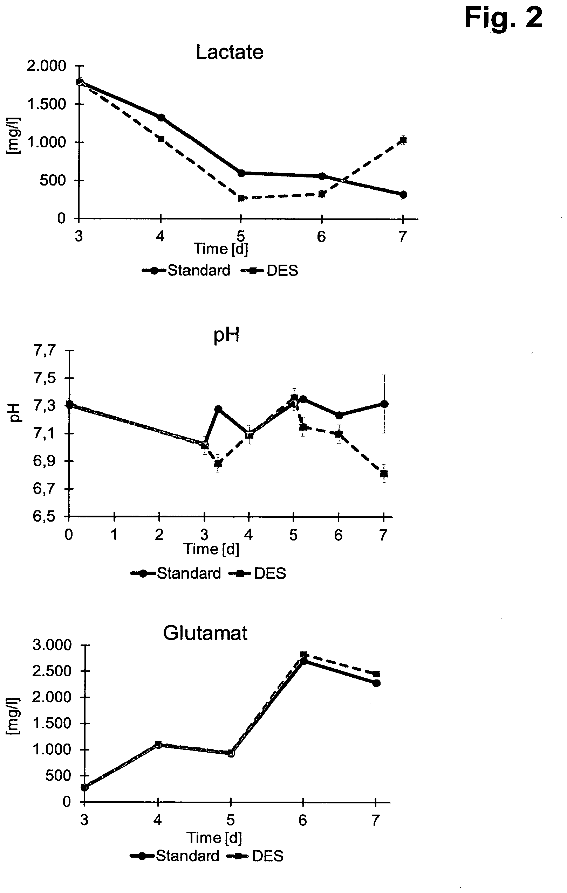

CHO-S cells are cultured according to the fed batch procedure described above. After measurements on day 10, the experiment is terminated because of low viability. Osmolality is only slightly higher in DES compared to Standard.
The basic metabolic rates are close to identical.
After feeding the pH is lower in DES compared to Standard because the DES is very acidic.
The results are also shown in Figures 1 and 2, whereby Figure 1 shows the viability and osmolality and Figure 2 shows the metabolites Lactate and Glutamate as well as the pH of the cell culture.

### b) Transfected CHO-S and CHO-K1 cell cultures producing a monoclonal antibody

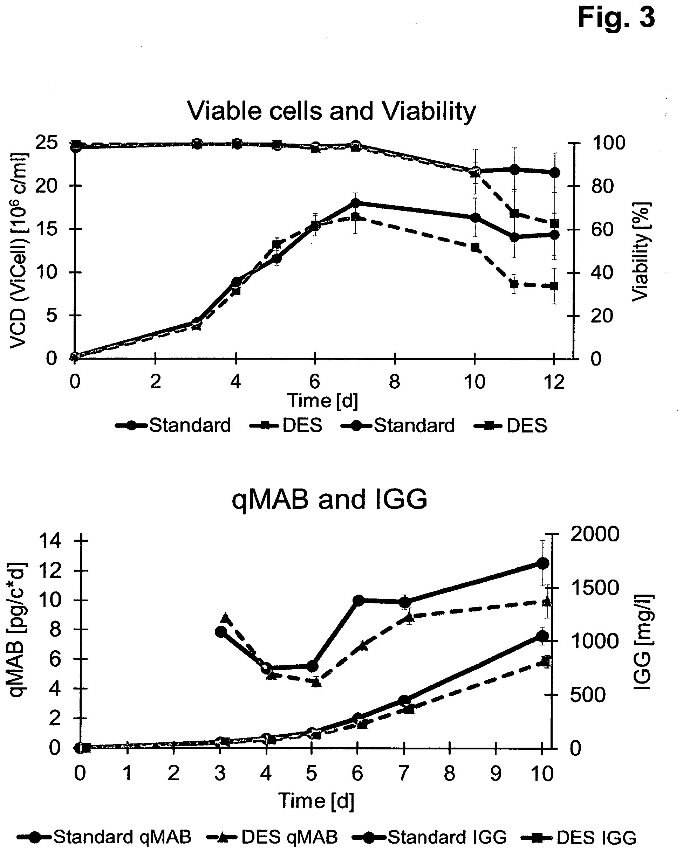

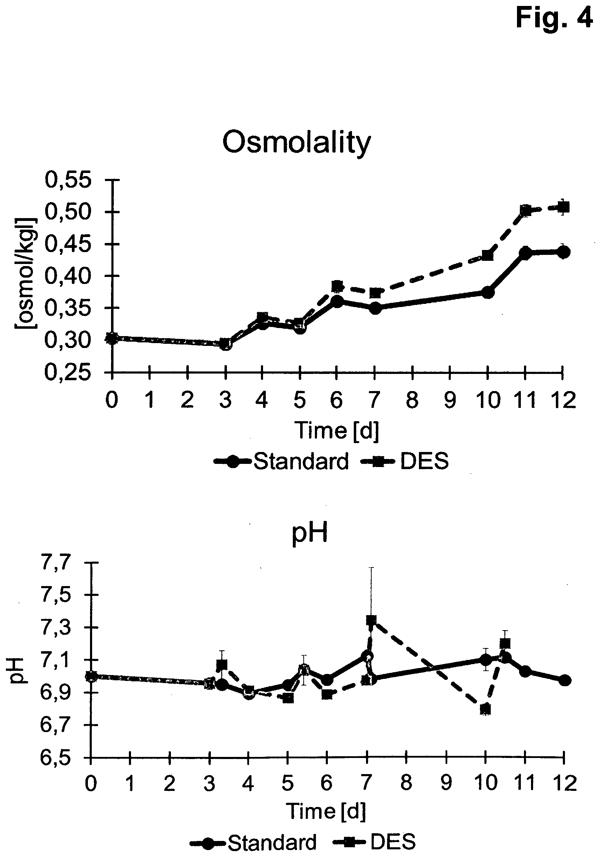

The experiments with transfected CHO-S and CHO-K1 cells demonstrate similar results as experiment 2a) (DES vs. Standard).
Comparison of typical aqueous Cys/Tyr feed compared to DES feed in fed-batch cultures with CHO-S cells is shown in Figures 3 and 4. Figure 3 shows the cellular specific monoclonal antibody production rate (qMAB) and absolute monoclonal antibody concentration (IGG) as well as the viable cell density and viability during fed-batch process till day 12. Error bars indicate one standard deviation.
Figure 4 shows the pH and the osmolality.
It can be seen that the liquid feed media according to the invention can be successfully applied in fed batch processes.

## Claims

1. A liquid feed medium comprising a deep eutectic solvent comprised of choline and/or betaine and/or 2-methyl-choline, acetylcholine, lactylcholine, propinoylcholine, buturylcholine, the methyl-, ethyl-, vinyl- or butyl-ether of choline and/or salts thereof and one or more amino acids and whereby the liquid feed medium comprises less than 50% of water (w/w).

2. A liquid feed medium according to claim 1, **characterized in that** it is in a liquid state of matter at 37 °C.

3. A liquid feed medium according to claim 1 or claim 2, **characterized in that** it comprises tyrosine and/or cysteine.

4. A liquid feed medium according to one or more of claims 1 to 3, **characterized in that** it comprises less than 10 % of water.

5. A liquid feed medium according to one or more of claims 1 to 4, **characterized in that** the liquid feed medium comprises other components which are not part of the deep eutectic solvent and which are dissolved in the deep eutectic solvent.

6. A process for cell culture comprising the following steps:
a) Providing a bioreactor with cells in a liquid cell culture medium
b) Adding to said liquid cell culture medium a liquid feed medium according to one or more of claims 1 to 5.

7. A process according to claim 8, **characterized in that** the liquid feed medium is added several times in the course of the process.

8. A process according to claim 6 or 7, **characterized in that** the liquid feed medium added in step b) has a temperature between 20 and 35°C.

9. A process according to one or more of claims 6 to 8, **characterized in that** the liquid feed medium added in step b) comprises choline and an amino acid.

10. A process according to one or more of claims 6 to 9, **characterized in that** the liquid feed medium added in step b) comprises other components which are not part of the deep eutectic solvent which are dissolved in the deep eutectic solvent.

11. A process according to one or more of claims 6 to 10, **characterized in that** the cells in the bioreactor are stem cells, eukaryotic cells, prokaryotic cells, bacteria, archaea, yeasts, fungi, insect cells or algae.

12. A process according to one or more of claims 6 to 11, **characterized in that** the liquid feed medium added in step b) comprises less than 10% (w/w) of water.

13. The use of a liquid feed medium according to one or more of claims 1 to 5 as a nutrient feed.

## Patentansprüche

1. Flüssiges Fütterungsmedium, enthaltend ein stark eutektisches Lösungsmittel, das aus Cholin und/oder Betain und/oder 2-Methylcholin, Acetylcholin, Lactylcholin, Propinoylcholin, Buturylcholin, dem Methyl-, Ethyl-, Vinyl- oder Butylether von Cholin und/oder Salzen davon und einer oder mehreren Aminosäuren besteht, und wobei das flüssige Fütterungsmedium weniger als 50% Wasser (Gew./Gew.) enthält.

2. Flüssiges Fütterungsmedium nach Anspruch 1, **dadurch gekennzeichnet, dass** es bei 37°C in einem flüssigen Materiezustand vorliegt.

3. Flüssiges Fütterungsmedium nach Anspruch 1 oder Anspruch 2, **dadurch gekennzeichnet, dass** es Tyrosin und/oder Cystein enthält.

4. Flüssiges Fütterungsmedium nach einem oder mehreren der Ansprüche 1 bis 3, **dadurch gekennzeichnet, dass** es weniger als 10% Wasser enthält.

5. Flüssiges Fütterungsmedium nach einem oder mehreren der Ansprüche 1 bis 4, **dadurch gekennzeichnet, dass** das flüssige Fütterungsmedium andere Komponenten enthält, die nicht Teil des stark eutektischen Lösungsmittels sind und die in dem stark eutektischen Lösungsmittel gelöst sind.

6. Verfahren zur Zellkultur, enthaltend die folgenden Schritte:
a) Bereitstellen eines Bioreaktors mit Zellen in einem flüssigen Zellkulturmedium
b) Hinzufügen zu dem flüssigen Zellkulturmedium eines flüssigen Fütterungsmediums nach einem oder mehreren der Ansprüche 1 bis 5.

7. Verfahren nach Anspruch 8, **dadurch gekennzeichnet, dass** das flüssige Fütterungsmedium mehrmals im Verlauf des Verfahrens zugegeben wird.

8. Verfahren nach Anspruch 6 oder 7, **dadurch gekennzeichnet, dass** das in Schritt b) zugegebene flüssige Fütterungsmedium eine Temperatur zwischen 20 und 35°C hat.

9. Verfahren nach einem oder mehreren der Ansprüche 6 bis 8, **dadurch gekennzeichnet, dass** das in Schritt b) zugegebene flüssige Fütterungsmedium Cholin und/oder eine Aminosäure enthält.

10. Verfahren nach einem oder mehreren der Ansprüche 6 bis 9,**dadurch gekennzeichnet, dass** das in Schritt b) zugegebene flüssige Fütterungsmedium andere Komponenten enthält, die nicht Teil des stark eutektischen Lösungsmittels sind, die in dem stark eutektischen Lösungsmittel gelöst sind.

11. Verfahren nach einem oder mehreren der Ansprüche 6 bis 10, **dadurch gekennzeichnet, dass** die Zellen im Bioreaktor Stammzellen, eukaryotische Zellen, prokaryotische Zellen, Bakterien, Archaea, Hefen, Pilze, Insektenzellen oder Algen sind.

12. Verfahren nach einem oder mehreren der Ansprüche 6 bis 11, **dadurch gekennzeichnet, dass** das in Schritt b) zugegebene flüssige Fütterungsmedium weniger als 10% (Gew./Gew.) Wasser enthält.

13. Verwendung eines flüssigen Fütterungsmediums nach einem oder mehreren der Ansprüche 1 bis 5 als Nährstofffutter.

## Revendications

1. Milieu d'alimentation liquide, comprenant un solvant eutectique profond constitué de choline et/ou de bétaïne et/ou de 2-méthylcholine, d'acétylcholine, de lactylcholine, de propinoylcholine, de buturylcholine, de méthyl-, éthyl-, vinyl- ou butyléther de choline et/ou des sels de ceux-ci et d'un ou plusieurs acides aminés, le milieu d'alimentation liquide comprenant moins de 50% d'eau (p/p).

2. Milieu d'alimentation liquide selon la revendication 1, **caractérisé en ce qu'**il se trouve dans un état liquide de matière à 37°C.

3. Milieu d'alimentation liquide selon la revendication 1 ou la revendication 2, **caractérisé en ce qu'**il comprend de la tyrosine et/ou de la cystéine.

4. Milieu d'alimentation liquide selon l'une ou plusieurs parmi les revendications 1 à 3, **caractérisé en ce qu'**il comprend moins de 10% d'eau.

5. Milieu d'alimentation liquide selon l'une ou plusieurs parmi les revendications 1 à 4, **caractérisé en ce que** le milieu d'alimentation liquide comprend d'autres composants qui ne font pas partie du solvant eutectique profond et qui sont solubilisés dans le solvant eutectique profond.

6. Procédé de culture cellulaire, comprenant les étapes suivantes :
a) l'alimentation d'un bioréacteur en cellules dans un milieu de culture cellulaire liquide ;
b) l'addition, audit milieu de culture cellulaire liquide, d'un milieu d'alimentation liquide selon l'une ou plusieurs parmi les revendications 1 à 5.

7. Procédé selon la revendication 8, **caractérisé en ce que** le milieu d'alimentation liquide est ajouté plusieurs fois au cours du procédé.

8. Procédé selon la revendication 6 ou 7, **caractérisé en ce que** le milieu d'alimentation liquide ajouté dans l'étape b) possède une température comprise entre 20 et 35°C.

9. Procédé selon l'une ou plusieurs parmi les revendications 6 à 8, **caractérisé en ce que** le milieu d'alimentation liquide ajouté dans l'étape b) comprend de la choline et un acide aminé.

10. Procédé selon l'une ou plusieurs parmi les revendications 6 à 9, **caractérisé en ce que** le milieu d'alimentation liquide ajouté dans l'étape b) comprend d'autres composants qui ne font pas partie du solvant eutectique profond et qui sont solubilisés dans le solvant eutectique profond.

11. Procédé selon l'une ou plusieurs parmi les revendications 6 à 10, **caractérisé en ce que** les cellules dans le bioréacteur sont des cellules souches, des cellules eucaryotes, des cellules procaryotes, des bactéries, des archéobactéries, des levures, des champignons, des cellules d'insectes ou des algues.

12. Procédé selon l'une ou plusieurs parmi les revendications 6 à 11, **caractérisé en ce que** le milieu d'alimentation liquide ajouté dans l'étape b) comprend moins de 10% (p/p) d'eau.

13. Utilisation d'un milieu d'alimentation liquide selon l'une ou plusieurs parmi les revendications 1 à 5, comme alimentation nutritive.
